# EUROPÄISCHE PATENTANMELDUNG

(11) **EP 2 325 538 A1**
(43) Veröffentlichungstag der Anmeldung: **25.05.2011**
(21) Anmeldenummer: 09075510.9
(22) Anmeldetag: 18.11.2009
(51) Int. Cl.: F16L 33/28, F16L 33/30, A61F 2/06

(54) **Vorrichtung mit wenigstens einem Hohlelement zur Leitung eines Fluids und mit einem Verbindungselement**

(71) Anmelder: Berlin Heart GmbH, 12247 Berlin (DE)
(72) Erfinder: Göllner, Manfred, 13086 Berlin (DE); Müller, Johannes, 10717 Berlin (DE)
(74) Vertreter: Pfenning, Meinig & Partner GbR

(57) **Zusammenfassung**

Bei einer Vorrichtung mit wenigstens einem ersten Hohlelement (1,2,11,20) zur Leitung eines Fluids mit einem Verbindungselement (3,25) ist, um eine einfache und zuverlässige Verbindung mit einem zweiten Hohlelement zu ermöglichen und zu erleichtern, ein Schnappelement (3a,3d,12a,24a) des Verbindungselementes mit einer Rastfläche, die in einem Verbindungszustand mit dem zweiten Hohlelement an einer Anschlagfläche (4a,13a,23a) anliegt, als radial aufweitbarer Schnappring ausgeführt.

## Beschreibung

Die Erfindung bezieht sich auf eine Vorrichtung mit einem Hohlelement, beispielsweise einem Rohr, einem Schlauch oder ähnlichen Bauteilen zur Leitung eines Fluids, beispielsweise eines Gases oder einer Flüssigkeit, und mit einem Verbindungselement zur Verbindung des Hohlelementes mit wenigstens einem weiteren Hohlelement.

In der Technik stellt sich oft die Aufgabe, verschiedene Rohre, Schläuche oder sonstige Leitungen, in denen Fluide geführt werden, miteinander zuverlässig, fest und dicht zu verbinden.

Hierzu sind bereits viele mehr oder weniger aufwendige Lösungen bekannt, die unter bestimmten Umgebungsbedingungen realisierbar sind. Dabei erfordern besondere Umgebungsbedingungen wie beispielsweise schwierige Zugänglichkeit der Verbindungsstelle oder auch die geringe Baugröße der zu schaffenden Verbindung besondere Lösungen. Als besondere Anforderung kann auch hinzukommen, dass trotz der Dichtigkeit der Verbindung diese noch beweglich, beispielsweise um ihre Längsachse drehbar sein soll.

Besonders anspruchsvolle Randbedingungen stellen sich auf dem Gebiet der Medizintechnik, wenn beispielsweise derartige Hohlelemente in Form von Schläuchen oder Kanülen vorliegen, in denen Körperflüssigkeiten, körperverträgliche Flüssigkeiten oder andere Flüssigkeiten innerhalb eines Körpers transportiert werden sollen. Solche Verbindungen sind beispielsweise bei chirurgischen Eingriffen schwer zugänglich und müssen einerseits verlässlich dicht in kurzer Zeit herstellbar sein, andererseits platzsparend und an individuelle Körpermaße anpassbar sein.

Es ist bereits bekannt, derartige Verbindungen als Pressverbindungen mit radialer Dichtung herzustellen, wie beispielsweise in dem deutschen Gebrauchsmuster DE 202 18 931 U1 bei einem Kanülenadapter für weiche Katheter angesprochen.

Aus der deutschen Offenlegungsschrift DE 10 2006 035 257 A1 ist beispielsweise eine Kathetersteckverbindung bekannt.

Schließlich sind auch Verbindungsstücke zum Anschluss von Hohlelementen an Blutgefäße bekannt, wie beispielsweise das aus der kanadischen Patentschrift CA 2376118 A1 bekannte T-förmige Verbindungsstück, das in ein Blutgefäß eingebracht werden kann. Schließlich ist aus der WO 2004/001272 A1 eine Verbindungsvorrichtung für Kanülen mit einem Überwurf bekannt, der eine Rastverbindung zwischen den zu verbindenden Kanülen ermöglicht.

Vor dem Hintergrund des Standes der Technik liegt der vorliegenden Erfindung die Aufgabe zugrunde, eine Vorrichtung der eingangs genannten Art konstruktiv besonders einfach zu realisieren und dabei eine besonders einfache Handhabbarkeit zu erreichen.

Die Aufgabe wird durch die Erfindung mit den Merkmalen des Patentanspruchs 1 gelöst.

Dazu ist an dem ersten Hohlelement, das mit einem zweiten Hohlelement zur Leitung eines Fluids verbindbar sein oder verbunden werden soll, ein Verbindungselement vorgesehen, das ein verformbares Schnappelement mit einer Rastfläche aufweist, welche derart eingerichtet ist, dass sie in einem Verbindungszustand der beiden Hohlelemente an einer mit dem zweiten Hohlelement verbundenen Anschlagfläche anliegt. Das Schnappelement ist erfindungsgemäß als radial aufweitbarer Schnappring ausgebildet. Die Rastfläche kann demgemäß eine ganz durchgehende oder sektorförmig unterbrochene Kreisringfläche aufweisen.

Ein solcher Schnappring hat gegenüber einzelnen Schnappelementen beispielsweise in Form von Schnapphaken mit Rastnasen den Vorteil, einfacher herstellbar zu sein, beispielsweise in einem Spritzgussverfahren, wobei einzelne Rasthaken abbrechen oder abreißen können, was insbesondere bei der Verwendung in der chirurgischen Technik innerhalb eines Patientenkörpers negative Folgen haben kann. Ein Schnappring kann zudem eine sicherere Verbindung gewährleisten, da die Anschlagflächen größer sind, und es können in einfacher Weise höhere Rastkräfte, d. h. nach innen gerichtete Haltekräfte des Schnapprings gewährleistet werden, die gegen die Aufweitung des Schnapprings wirken und damit die hergestellte Verbindung sichern.

Außerdem ist die Schnappverbindung mit einem Schnappring um die Längsachse einfach drehbar, ohne dass das Verhaken einzelner Rastnasen durch deren Verbiegung oder durch das Hängenbleiben an Unebenheiten am Gegenstück zu befürchten ist. Dennoch kann die entsprechende Rastverbindung lösbar ausgestaltet sein.

Zum Herstellen der Schnappverbindung kann an dem Schnappring eine Einlaufschräge in Form eines Konus vorgesehen sein, die beim Aufschieben auf eine Gegenfläche die Aufweitung des Schnapprings vor dem Einschnappen an der Anschlagfläche bewirkt.

Vorteilhaft ist der Schnappring allein durch Materialelastizität elastisch aufweitbar. Er kann zu diesem Zweck aus einem Elastomer, wie beispielsweise einem Silikonelastomer hergestellt sein. Aus diesem Material kann das gesamte Verbindungselement und gegebenenfalls auch das erste und/oder das zweite Hohlelement bestehen. Damit ist eine einfache Verformbarkeit der angesprochenen Elemente auch zum Zwecke der Anpassung an die anatomischen Gegebenheiten bei einer Operation besonders einfach möglich.

Es kann jedoch auch vorgesehen sein, dass eines der Hohlelemente als starres Rohr und das andere Hohlelement als flexible Kanüle ausgebildet ist.

Der Schnappring kann grundsätzlich zusätzlich zu seiner materialbedingten Elastizität auch Radialschlitze in seiner Längsrichtung, die parallel zu seiner Symmetrieachse verläuft, aufweisen, die die radiale Aufweitung erleichtern.

Grundsätzlich ist es jedoch auch vorteilhaft, den Schnappring ungeschlitzt herzustellen, um glatte Konturen, eine Zylindersymmetrie und damit eine gute Drehbarkeit um die Längsachse zu erreichen.

Die Handhabbarkeit der erfindungsgemäßen Vorrichtung wird dadurch besonders einfach, dass wenigstens der Schnappring radial nach außen über andere Teile des Verbindungselementes umbördelbar ist. Damit lässt sich das Verbindungselement mit aufgeweitetem Schnappring in eine geeignete Position zu dem zweiten Hohlelement bringen und dann lässt sich die Umbördelung durch Streckung des Verbindungselementes rückgängig machen, um den Schnappring an die Anschlagfläche des zweiten Hohlelementes anzulegen.

Dies ist insbesondere dann vorteilhaft, wenn der Schnappring in der umgebördelten Position stabilisiert ist. In diesem Fall kann zur Vorbereitung der Operation bzw. des Verbindungsvorgangs bereits der Schnappring umgebördelt werden, z. B. außerhalb des Patientenkörpers, um dann mit geringem Aufwand effizient während der Operation eine entsprechende Verbindung durch Einschnappen des Schnapprings herstellen zu können. Hierzu ist dann beispielsweise nur ein leichtes Schieben gegen den Schnappring notwendig, um das Zurückklappen des Schnapprings und das Rückgängigmachen der Umbördelung zu erreichen.

Um die Stabilisierung des Schnapprings in der umgebördelten Position auf einfache Weise zu erreichen, kann gemäß der Erfindung vorgesehen sein, dass das Verbindungselement in einem axial begrenzten Bereich eine Wandschwächung aufweist. Diese kann dadurch realisiert sein, dass in dem entsprechenden, axial begrenzten Bereich Ausnehmungen in dem Verbindungselement an dessen Umfang oder Ausnehmungen in der Wand des Verbindungselementes vorgesehen sind, die den Querschnitt schwächen. Im einfachsten Fall kann eine innen oder außen umlaufende Nut an dem Verbindungselement vorgesehen sein. Das Verbindungselement kann alternativ oder zusätzlich in dem axialen Abschnitt auch aus einem weniger steifen Material bestehen als in den übrigen Bereichen.

Vorteilhaft kann gemäß der Erfindung vorgesehen sein, dass das Verbindungselement zylindrisch ausgebildet ist und an beiden Enden jeweils einen radial aufweitbaren Schnappring aufweist, der mit je einer Anschlagfläche an jeweils einem Hohlelement zusammenwirkt. Damit kann das Verbindungselement sowohl mit dem ersten Hohlelement als auch mit einem zweiten Hohlelement durch Einschnappen jeweils eines Schnappringes an einer Anschlagfläche verbunden werden. Damit ergibt sich eine verbesserte Drehbarkeit der Gesamtanordnung um die Längsachse, und es ergeben sich mehr Möglichkeiten zur Handhabung, da das Verbindungselement wahlweise auf das erste oder zweite Hohlelement zuerst aufschnappbar ist und dann während einer Operation auf das jeweils andere Hohlelement aufgeschnappt werden kann.

Alternativ dazu kann das Verbindungselement auch mit dem ersten oder zweiten Hohlelement einstückig zusammenhängen oder durch Kleben oder Schweißen mit diesem verbunden sein. Dies kann die Handhabbarkeit insofern erleichtern, als jeweils alle für die Herstellung einer Verbindung notwendigen Teile ohne zusätzliche Maßnahmen an einer Verbindungsstelle unbedingt verfügbar sind. Es ist in diesem Fall auch eine Schnappverbindung weniger herzustellen, da diese werkseitig bei der Herstellung durch eine feste Verbindung ersetzt ist, so dass der Gesamtaufwand bei der Herstellung einer Verbindung vor Ort reduziert ist. Das Verbindungsstück kann beispielsweise in einem einzigen Spritzgießvorgang mit dem ersten Hohlelement einstückig zusammen hergestellt sein.

Die Erfindung bezieht sich zudem auf eine Vorrichtung der oben beschriebenen Art, bei der das erste oder zweite Hohlelement als Blutgefäßanschlussstück ausgebildet ist mit einem radial nach außen ragenden Anschlussflansch an seinem dem Schnappelement entgegengesetzten Ende. Durch ein derartiges Blutgefäßanschlussstück soll beispielsweise eine einfache und zuverlässige Verbindung zwischen einer Kanüle und einem Blutgefäß herstellbar sein. Es ist üblich, hierzu einen Kanülenkopf (Graft) an ein Gefäß anzunähen. Solche Verbindungen funktionieren bisher nicht optimal, da die Geometrie der entsprechenden Bauelemente nicht optimal auf die anatomischen Gegebenheiten abgestimmt oder abstimmbar sind. Die vorliegende Erfindung soll in diesem Zusammenhang besonders einfach nachträgliche Lagekorrekturen ermöglichen und durch Vor-Auswahl der geometrischen Parameter eine möglichst gute Anpassung an die anatomischen Gegebenheiten erlauben. Außerdem soll ein derartiges Blutgefäßanschlussstück einfach mit weiteren Hohlelementen verbindbar sein, ohne dass nach der Verbindung mit einem Blutgefäß noch größere Kräfte auf das Blutgefäßanschlussstück wirken oder dieses in wesentlichem Ausmaß bewegt werden muss.

Für eine optimierte Dichtung an einem Blutgefäß ist gemäß der Erfindung vorgesehen, dass der Anschlussflansch eine konvexe Dichtfläche aufweist. Diese Dichtfläche kann im einfachsten Fall einen Flächenabschnitt der Oberfläche eines Hohl-Zylinders bilden, indem sie in einer Richtung, der Längsrichtung des Blutgefäßes, gerade verläuft und in einer zweiten Richtung im Querschnitt kreisförmig. Die Anschlussfläche kann jedoch in der zweiten Richtung auch einen nicht kreisförmigen runden Querschnitt aufweisen, beispielsweise einen elliptischen oder anderweitig abgerundeten Querschnitt, der entsprechenden anatomisch vorliegenden realen Querschnitten von Blutgefäßen entspricht.

An seinem dem Anschlussflansch entgegengesetzten Ende kann das Blutgefäßanschlussstück vorteilhaft eine kegelförmige Dichtfläche aufweisen. An dieser kann ein Gegenstück eines weiteren Hohlelementes, das mit dem Blutgefäßanschlussstück zu verbinden ist, anliegen. Dabei kann die Dichtfläche des Blutgefäßanschlussstückes eine kegelförmige Innenkontur oder eine kegelförmige Außenkontur aufweisen, wobei die Gegenfläche des weiteren Hohlelementes jeweils eine entsprechend geformte Gegenfläche aufweist.

Es kann auch vorteilhaft vorgesehen sein, dass das Blutgefäßanschlussstück an seinem dem Anschlussflansch entgegengesetzten Ende eine kugelflächenförmige Führungsfläche und /oder Dichtfläche aufweist. Dazu kann das Blutgefäßanschlussstück an seinem Ende beispielsweise eine hohlkugelförmige Innenfläche aufweisen, in die eine Teilkugelaußenfläche eines Gegenstücks eines weiteren Hohlelementes eingelegt ist, oder das Blutgefäßanschlussstück kann eine Teilkugelaußenfläche aufweisen, die in eine entsprechende Hohlkugelteilfläche eines Gegenstücks dichtend hineinragt.

Durch diese Konstruktion ist eine allseitige Schwenkbarkeit und Verdrehbarkeit des Blutgefäßanschlussstücks gegenüber einem mit diesem zu verbindenden Hohlelement gewährleistet.

Wenn die Schwenkbarkeit in engen Winkelgrenzen bleibt, kann die Verbindung durch ein Verbindungsstück der oben beschriebenen Art mit einem oder mehreren Schnappringen an seinen Enden zuverlässig gesichert und besonders einfach hergestellt werden. Dabei kann es sich als besonders vorteilhaft erweisen, wenn das Verbindungsstück insgesamt elastisch ist, so dass es beim Schwenken der beiden zu verbindenden Hohlelemente gegeneinander biegbar ist, ohne dass der jeweilige Schnappring sich wesentlich verformt.

Die erfindungsgemäße Vorrichtung ermöglicht ein einfaches und zuverlässiges Herstellen einer Verbindung zwischen mehreren Hohlelementen, insbesondere medizinischen Kanülen, wobei der Herstellungsaufwand wegen der konstruktiv einfachen Realisierung gering bleibt. Auch die Verwendung eines Blutgefäßanschlussstücks der beschriebenen Art an sich ohne ein Verbindungsstück mit einem Schnappring kann für sich schon vorteilhaft sein und bestimmte technische Aufgaben lösen. Dabei kann bei einem solchen Anschlussstück entweder eine kugelige Dichtflächenkombination wie oben beschrieben verwendet werden, oder es kann eine Biegbarkeit des Anschlussstücks durch entsprechende Materialauswahl oder Verwendung eines Wellschlauches zumindest auf einem Teil der Länge des Anschlussstücks realisiert werden. Es kann auch in Betracht kommen, eine Mehrzahl entsprechender Anschlussstücke mit unterschiedlichen vorgegebenen Kröpfungen bereitzuhalten.

Im Folgenden wird die Erfindung anhand eines Ausführungsbeispiels in einer Zeichnung gezeigt und nachfolgend beschrieben.

Dabei zeigt
- Fig. 1: in einem Längsschnitt ein erstes und ein zweites Hohlelement sowie ein mit dem ersten Hohlelement verbundenes Verbindungselement,
- Fig. 2: die Anordnung aus Fig. 1 mit umgebördeltem Verbindungselement,
- Fig. 3: die Anordnung aus Fig. 1, wobei die Hohlelemente miteinander verbunden sind,
- Fig. 4: im Längsschnitt eine Verbindung zwischen einem Pumpenrohr und einer Kanüle,
- Fig. 5: im Längsschnitt das Ende einer Kanüle und im Querschnitt ein Blutgefäß,
- Fig. 6: in dreidimensionaler Darstellung das Ende einer Kanüle mit einem Blutgefäßanschlussstück und einem Blutgefäß,
- Fig. 7: in einem Schnitt eine Kanüle, ein Blut-gefäßanschlussstück und ein Blutgefäß,
- Fig. 8: die Anordnung aus Fig. 7 in einer drei-dimensionalen Darstellung,
- Fig. 9: die Anordnung aus Fig. 7 im verbundenen Zustand,
- Fig. 10: eine Ansicht eines Blutgefäßanschluss-stücks, das auf ein Blutgefäß aufgesetzt ist,
- Fig. 11: die Anordnung aus Fig. 10, wobei das Blut-gefäßanschlussstück gegenüber der in Fig. 10 gezeigten Position gedreht ist,
- Fig. 12: eine Anordnung eines Kanülenendes, das mittels eines Verbindungselements mit einem Blutgefäßanschlussstück verbunden ist, im Querschnitt, wobei zwischen dem Blutgefäß-anschlussstück und dem Kanülenende ein Kugelgelenk vorgesehen ist.

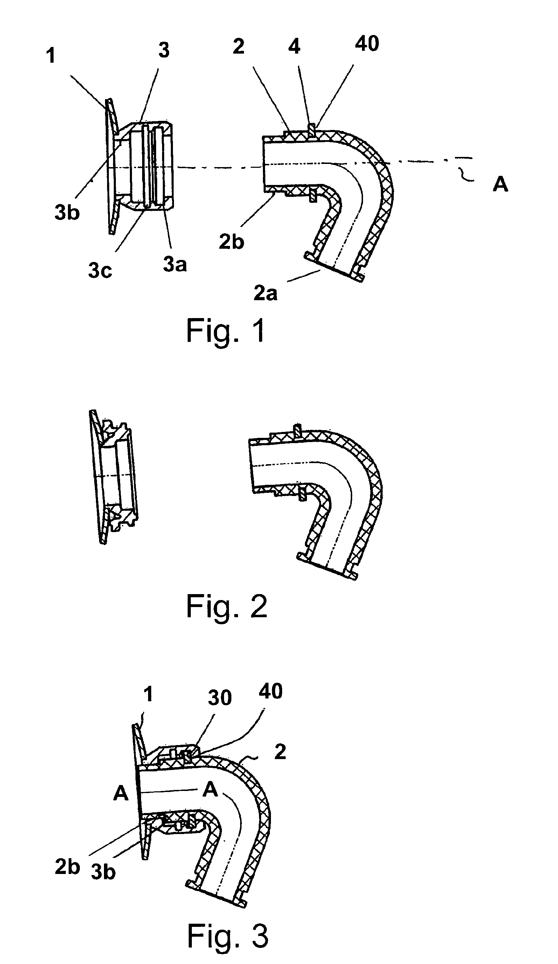

Fig. 1 zeigt im linken Teil als ein Beispiel für ein Hohlelement, das von einem Fluid durchflossen wird, einen sogenannten Nahtring 1, der beispielsweise an ein Herz angenäht werden kann und der mit einem Ende 2b einer Einlasskanüle 2 zu verbinden ist, an deren anderem Ende 2a eine Blutpumpe angeordnet sein kann.

Anstelle des Nahtrings 1 kann jedoch auch zu anderen Zwecken eine weitere Kanüle vorgesehen sein.

Der Nahtring 1 trägt einen aus Silikon hergestellten elastischen Überwurf 3, der das Verbindungselement darstellt und der mit dem Nahtring 1 verklebt ist. Der Nahtring 1 kann mittels des Verbindungsstücks 3 mit der Kanüle 2 verbunden werden. Dazu wird zunächst, wie in Fig. 2 dargestellt, der Überwurf 3 im Bereich der Innennut 3c nach außen umgebördelt. In dieser Position wird das Verbindungselement durch die elastische Aufweitung seines Endes mit der Anschlagfläche 3a und dessen entsprechendes Bestreben, sich radial zusammenzuziehen, selbständig stabilisiert. In dieser Position kann während der Operation der Nahtring mit dem so vorbereiteten Überwurf gut gehandhabt werden.

Die Kanüle 2 wird mit ihrem Ende 2b in den Überwurf 3 eingeschoben, wobei der engste Bereich 3b des Überwurfs vorteilhaft einen geringeren Durchmesser aufweist als das Ende 2b der Kanüle, so dass hier durch elastischen Druck in radialer Richtung eine Dichtung erzielt wird. Nach dem Einschieben der Kanüle 2 in den Überwurf 3 wird der umgebördelte Teil des Überwurfs 3 wieder gestreckt, so dass die Anschlagfläche 3a des Überwurfs 3 mit der Anschlagfläche 4a des in die Kanüle 2 eingesetzten starren Rings 4 zusammenwirkt. Damit sind der Nahtring 1 und die Kanüle 2 auch in axialer Richtung (Achse A) zusammengehalten. Zudem kann der Kontakt zwischen den Anschlagflächen 3a, 4a auch eine Dichtung in axialer Richtung bewirken.

Der Ring 4 kann in eine Nut der Kanüle 2 eingeklebt oder in diese elastisch eingeschnappt sein. Der Ring 4 kann dazu als starrer Ring beispielsweise aus Metall in geschlitzter Form oder auch als elastischer, durchgehender Ring ausgeführt sein.

Die zusammengefügte Stellung des Nahtrings 1 und der Kanüle 2 ist in der Fig. 3 dargestellt. Dort ist auch zu erkennen, dass der Bereich 2b der Kanüle mit dem Bereich 3b des Überwurfs 3 kraftschlüssig zusammenpasst.

Ebenso ist die Anschlagfläche 3a des Überwurfs an die Anschlagfläche 4a des Rings 4 in axialer Richtung angepresst.

Es ist zu bemerken, dass das Überwurfelement 3 mit dem Nahtring 1 auch einstückig zusammenhängen könnte oder dass der Nahtring 1 einen Stutzen mit einem Ring ähnlich dem Ring 4 aufweisen könnte, mit dem der Überwurf in gleicher Weise zusammenwirken kann wie auf der gegenüberliegenden Seite mit der Kanüle 2.

Die Innennut 3c in dem Überwurf ist derart gestaltet, dass die verbleibende Wandstärke, wie in Fig. 1 auf der linken Seite zu erkennen, deutlich geschwächt ist, so dass sich der Überwurf 3 an dieser Stelle am einfachsten nach außen umlegen/umbördeln lässt. Dabei ist hilfreich, wenn der umzubördelnde Teil des Überwurfs 3 wenigstens stellenweise einen geringeren Innendurchmesser aufweist als der ,Außendurchmesser des Überwurfs im Bereich der Innennut 3c, so dass der Überwurf sich in dem umgebördelten Zustand besonders gut selbst stabilisiert.

Der Ring 4, der die Anschlagfläche 4a im Bereich der Kanüle 2 zum Zusammenwirken mit der Anschlagfläche 3a des Überwurfs bildet, kann auch einstückig mit der Kanüle hergestellt sein.

Die Maße des Überwurfs und das Material des Rings 4 können auch derart gestaltet sein, dass wenigstens eines dieser Elemente elastisch deformierbar ist und dass im zusammengefügten Zustand der beiden Hohlelemente ein axialer Zug auf dem Überwurf 3 lastet, so dass im Bereich der Anschlagflächen 3a, 4a eine axiale Dichtung gegeben ist. Diese kann zusätzlich oder alternativ zu der beschriebenen radialen Dichtung der zusammengefügten Bereiche 2b, 3b bestehen.

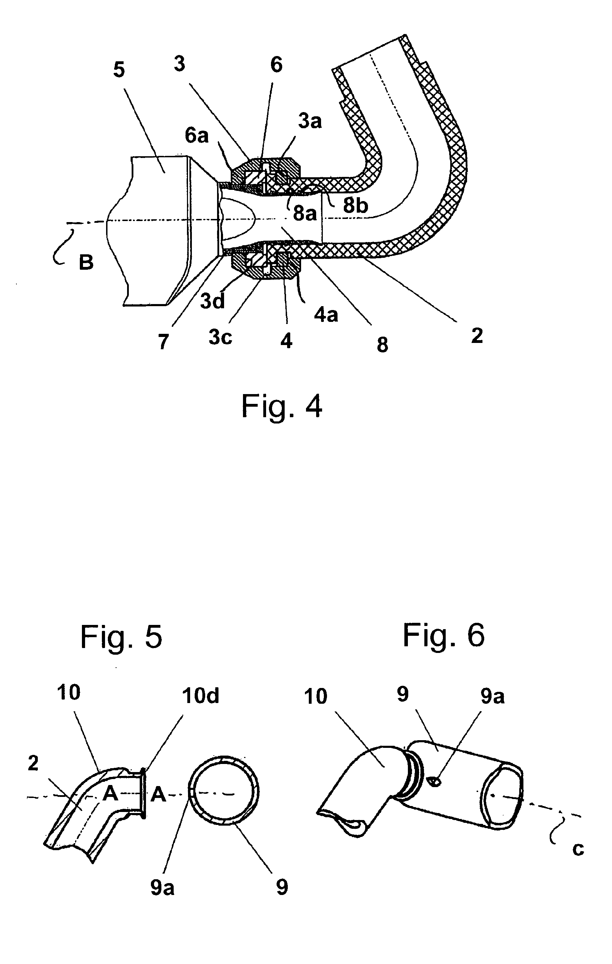

Fig. 4 zeigt in einem Längsschnitt den Anschluss einer Kanüle 2 an das Pumpenrohr 7 einer Blutpumpe 5. Die Kanüle 2 weist, wie in den Fign. 1-3 beschrieben, einen eingefügten Ring 4 auf, der über eine Anschlagfläche 4a verfügt, die mit einer entsprechenden Anschlagfläche 3a eines Überwurfs 3 zusammenwirkt. Der Überwurf 3 ist im Falle der Fig. 4 über einen Ring 6 gelegt, der mit dem Pumpenrohr 7 fest verbunden, beispielsweise verschweißt oder verklebt ist und der eine Anschlagfläche 6a aufweist, die mit einer Anschlagfläche 3d des Überwurfs 3 zusammenwirkt. Der Überwurf kann in diesem Sinn über die Innennut 3c sowohl zur Seite der Kanüle 2 hin umgebördelt werden, um dann auf einfache Weise mit dem Pumpenrohr verbunden zu werden, wie auch umgekehrt der Überwurf 3 zur Seite des Pumpenrohrs 7 hin umgebördelt werden kann, um dann einfach mit der Kanüle verbunden werden zu können.

Es kann aber auch vorgesehen sein, dass der Überwurf 3 im Bereich der Anschlagfläche 3d mit dem Ring 6 und/oder dem Pumpenrohr 7 fest verbunden, beispielsweise verklebt ist. Das Pumpenrohr 7 weist eine sogenannte Olive 8 auf, die als Rohrstutzen mit nach außen weisenden sogenannten Ringzacken ausgebildet ist. Diese Ringzacken sind im Längsschnitt sägezahnförmig ausgebildet und ringförmig um die Olive herum umlaufend, so dass sie sich als Ringe in das elastische Material der Kanüle 2 einprägen und somit eine radiale Dichtung bilden können.

Zwei Ringzacken sind in der Fig. 4 mit 8a, 8b bezeichnet. Trotz der elastischen und teilweise formschlüssigen Verbindung zwischen der Kanüle 2 und dem Pumpenrohr bzw. der Olive 8 ist die Anordnung um die Achse B drehbar, um bei der Zusammenfügung der Teile die Konstruktion an anatomische Gegebenheiten anpassen zu können.

Fig. 5 zeigt schematisch in einem Längsschnitt eine Kanüle 2 gemäß dem Stand der Technik, die über einen Kanülenkopf 10 mit einem im Querschnitt dargestellten Blutgefäß 9 verbunden werden soll. Dazu ist eine Stirnfläche 10d des Kanülenkopfs 10 vorgesehen, die an die äußere Form des Blutgefäßes 9 möglichst gut angepasst werden soll.

Die Fig. 6 zeigt die entsprechende an sich bekannte Anordnung in einer dreidimensionalen Darstellung, wobei in dem Blutgefäß 9 eine Öffnung 9a vorgesehen ist, die durch einen Chirurgen in Form eines Längsschnitts entlang der Längsachse C des Blutgefäßes 9 eingebracht werden kann. Die entsprechende Verbindung zwischen dem Kanülenkopf 10 und dem Blutgefäß 9 soll durch die Erfindung so ausgestaltet werden, dass eine elastisch dichtende Kontaktfläche besteht, die durch entsprechende Formung der Stirnfläche 10d geschaffen werden kann und die beispielsweise dreidimensional teilzylindrisch oder ähnlich ausgebildet sein kann.

Zudem sollte die Kanüle 2 um die Achse A gegenüber dem Blutgefäß drehbar sein, um die Anordnung individuell einrichten zu können.

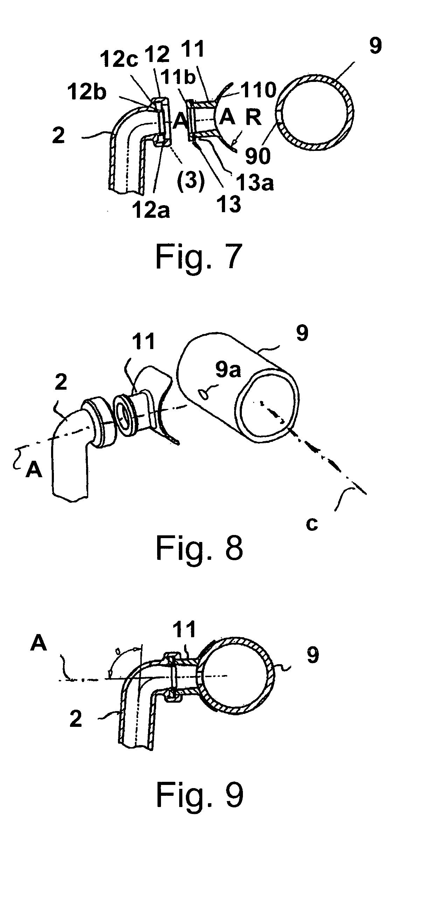

Zumindest die Kanüle 2 sollte mit dem Blutgefäß schnell und einfach verbunden und von diesem gegebenenfalls wieder getrennt werden können. Hierzu bietet sich die oben beschriebene erfindungsgemäße Verbindungsmethodik mithilfe eines umbördelbaren Verbindungsstücks an, die im Folgenden anhand der Fign. 7 ff. genauer erläutert wird.

Fig. 7 zeigt dazu ein Blutgefäßanschlussstück 11, das einen Anschlussflansch 11a aufweist. Der Anschlussflansch 11a ist beispielhaft teilzylindrisch dargestellt, womit er zur Verbindung mit einem im Querschnitt kreisförmigen Blutgefäß 9 vorbereitet ist. Andere Querschnitte des Anschlussflansches 11a sind ebenfalls denkbar. Diese können beispielsweise teilelliptisch ausgebildet sein und sich in ihrer Krümmung bzw dem Durchmesser R nach der Größe des Blutgefäßes 9 richten.

Das Blutgefäßanschlussstück 11 trägt an seinem der Kanüle 2 zugewandten Ende einen starren Ring 13, der dicht mit dem Blutgefäßanschlussstück verbunden, beispielsweise mit diesem einstückig hergestellt oder verschweißt oder verklebt ist und eine Anschlagfläche 13a trägt.

Am Ende der Kanüle 2 ist mit dieser das Verbindungsstück 3 einstückig verbunden. Dieses weist eine Anschlagfläche 12a auf, die mit der entsprechenden Anschlagfläche 13a des Rings 13 im verbundenen Zustand zusammenwirkt, wie in Fig. 9 dargestellt.

Die entsprechende Krempelnut ist mit 12c bezeichnet und schwächt das Verbindungsstück 3 an seiner Innenseite derart, dass die Umbördelung nach hinten zur Kanüle 2 hin einfach und selbststabilisierend ist. Mit 12b ist ein kegelförmiger Ansatz an der Kanüle 2 bezeichnet, der zum Ende der Kanüle 2 zuläuft und mit einem entsprechenden Hohlkegel 11b am Blutgefäßanschlussstück 11 im verbundenen Zustand zusammenwirkt, so dass bei dieser Verbindung eine Verkippung der Längsachsen der beiden Hohlelemente gegeneinander um einen bestimmten Betrag möglich ist, ohne dabei die Dichtwirkung der kegeligen Dichtelemente zu verlieren. Zudem ist die Verbindung um die Achse A problemlos drehbar, ohne dass die Dichtigkeit beeinträchtigt ist.

Zur Herstellung einer Verbindung zwischen der Kanüle und dem Blutgefäß 9 wird zunächst der Nahtring / das Blutgefäßanschlussstück 11 unter Nutzung des Anschlussflansches 11a an das Blutgefäß 9 angenäht und somit eine Dichtigkeit zwischen dem Blutgefäßanschlussstück und dem Gefäß hergestellt. Das Blut kann nun durch die Öffnung 9a aus- oder eintreten und das Blutgefäßanschlussstück 11 passieren. An dieses wird die Kanüle 2 dichtend angeschlossen, so dass über die Kanüle mit dem Blutgefäß 9 eine Blutpumpe verbunden werden kann.

Fig. 8 zeigt die Anordnung schematisch in einer dreidimensionalen Ansicht und Fig. 9 in einem Schnitt im zusammengefügten Zustand.

In der Fig. 9 ist auch der Kröpfungswinkel α der Kanüle erkennbar, der entweder durch Auswahl einer geeigneten Kanüle vorgewählt werden kann, oder die Kanüle kann biegsam ausgebildet sein, beispielsweise ganz oder abschnittsweise als Wellschlauch, um eine entsprechende Kröpfung erzielen zu können.

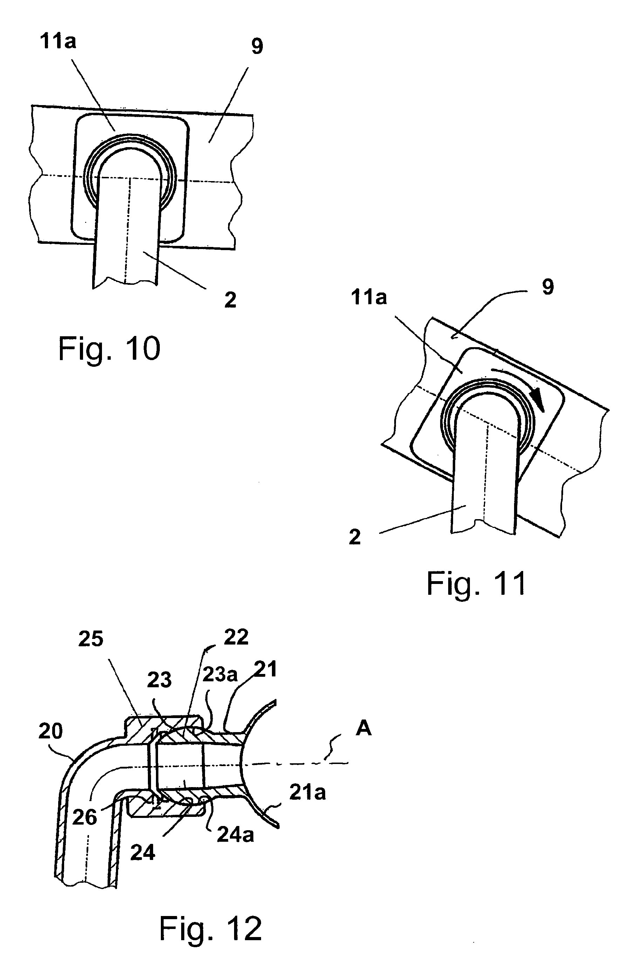

Fig. 10 zeigt in einer Draufsicht in Richtung der Achse A die mit dem Blutgefäßanschlussstück 11 verbundene Kanüle 2 vor dem Blutgefäß 9, mit dem der Flansch 11a des Blutgefäßanschlussstücks 11 vernäht ist.

Im Vergleich dazu zeigt Fig. 11 eine ähnliche Ansicht, bei der das Blutgefäß mitsamt dem Blutgefäßanschlussstück 11 ein Stück weit gegenüber der Kanüle 2 um die Achse A verdreht ist. Eine solche Verdrehung ist dank der Konstruktion gemäß der Erfindung ohne weiteres und ohne größeren Kraftaufwand realisierbar, so dass ein Abreißen der Vernähung des Anschlussflansches 11a an das Gefäß 9 nicht zu befürchten ist.

Sowohl die Kanüle 2 als auch das Blutgefäßanschlussstück mit dem Anschlussflansch 11a können beispielsweise aus einem Silikonelastomer bestehen, um die entsprechende Beweglichkeit zu erzielen.

Fig. 12 zeigt schließlich in einem Längsschnitt die Verbindung zwischen einer Kanüle 20 und einem Blutgefäßanschlussstück 21 mit einem Anschlussflansch 21a. Das Blutgefäßanschlussstück 21 weist ein kugelförmiges Endstück 22 mit einer teilkugelförmigen Dichtfläche 23 auf. Diese wirkt mit einer hohlkugelförmigen Dichtfläche 24 zusammen, die an einem Überwurf 25 angeordnet ist, der mit der Kanüle 20 einstückig verbunden ist. Der Überwurf 25 ist als Verbindungsstück ausgeführt, dessen hohlkugelförmige Dichtfläche 24 in dem Bereich 24a, der das kugelförmige Endstück 22 des Blutgefäßanschlussstückes hintergreift, als Anschlagfläche ausgebildet ist. Diese Anschlagfläche 24a wirkt mit dem entsprechenden Bereich 23a der hohlkugelförmigen Dichtfläche des Verbindungsstücks 25 derart zusammen, dass die Kanüle 20 einerseits und das Blutgefäßanschlussstück 21 andererseits entlang der Achse A in Figur 12 axial zusammengehalten werden. Das Verbindungsstück 25 kann, ebenso wie in den vorangehend beschriebenen Figuren dargestellt, auch nach außen zu der Kanüle 20 hin umgebördelt werden, um die Verbindung während einer Operation einfach durch Zurückschnappen des Überwurfes herstellen zu können. Dazu kann im Bereich, der gestrichelt dargestellt und mit 26 bezeichnet ist, eine Innennut an dem Verbindungsstück 25 vorgesehen sein. Das Verbindungsstück 25 kann ebenso wie die Kanüle 20 elastisch sein und aus einem Silikonelastomer bestehen.

Die dargestellte Ausführungsform hat die Eigenschaft, dass die Kanüle 20 und das Blutgefäßanschlussstück 21 einerseits um die Achse A gegeneinander verdrehbar und andererseits auch gegenüber dieser Achse gegeneinander zumindest ein Stück weit, beispielsweise um 5 bis 10 Grad, verkippbar sind. Die Dichtung zwischen den Kugelflächen 23, 24 wird dabei nicht gefährdet und auch die Anschlagflächen 23a, 24a bleiben in Funktion.

Die einfache Handhabung beim Zusammenfügen der Hohlelemente 20, 21 mittels des Verbindungselements 25 ist dabei ebenso wie bei den in Fign. 1-11 dargestellten Beispielen gegeben.

Es ist sinnvoll, die Kröpfung der Kanüle 20 auf die erforderlichen Gegebenheiten möglichst weitgehend einzustellen, damit die Verkippung zwischen der Kanüle 20 und dem Blutgefäßanschlussstück 21 beim tatsächlichen Gebrauch minimiert wird, um den Durchflusswiderstand dieser Verbindung und damit auch Verwirbelungen des diese durchströmenden Blutes zu minimieren, um Schädigungen bzw. Ablagerungen des Blutes dauerhaft zu vermeiden.

Das Verbindungselement 25 kann bei einer ähnlichen Ausführungsform anders als in der Fig. 12 auch mit dem Blutgefäßanschlussstück 21 fest verbunden sein und entsprechend eine kugelförmige Dichtfläche der Kanüle 20 elastisch umgreifen.

Die erfindungsgemäße Ausgestaltung der Verbindung zwischen zwei Hohlelementen erlaubt, ebenso wie die erfindungsgemäße Ausgestaltung eines Blutgefäßanschlussstücks eine besonders einfache Handhabung der entsprechenden Teile während einer Operation, wobei eine besonders geringe Fehleranfälligkeit verbunden ist mit einer hohen Zuverlässigkeit und Dichtigkeit der Verbindung.

## Patentansprüche

1. Vorrichtung mit wenigstens einem ersten Hohlelement (1,2,11,20) zur Leitung eines Fluids und mit einem Verbindungselement (3,25), welches ein verformbares Schnappelement aufweist mit einer Rastfläche (3a,3d,12a,24a), die in einem Verbindungszustand an einer mit
einem zweiten Hohlelement verbundenen Anschlagfläche (4a,13a,23a) anliegt, **dadurch gekennzeichnet, dass** das Schnappelement ein radial aufweitbarer Schnappring ist.

2. Vorrichtung nach Anspruch 1, **dadurch gekennzeichnet, dass** der Schnappring durch Materialelastizität elastisch aufweitbar ist.

3. Vorrichtung nach Anspruch 1 oder 2, **dadurch gekennzeichnet, dass** der Schnappring ungeschlitzt umlaufend ausgebildet ist.

4. Vorrichtung nach Anspruch 1, 2 oder 3, **dadurch gekennzeichnet, dass** wenigstens der Schnappring radial nach außen über andere Teile des Verbindungselementes (3,25) umbördelbar ist.

5. Vorrichtung nach Anspruch 4, **dadurch gekennzeichnet, dass** der Schnappring in der umgebördelten Position stabilisiert ist.

6. Vorrichtung nach einem der Ansprüche 1 bis 5,
**dadurch gekennzeichnet, dass** das Verbindungselement(3,25) in einem axial begrenzten Bereich eine Wandschwächung (3c) aufweist.

7. Vorrichtung nach Anspruch 1 oder einem der folgenden, **dadurch gekennzeichnet, dass** das Verbindungselement (3,25) zylindrisch ausgebildet ist und an beiden Enden jeweils einen radial aufweitbaren Schnappring aufweist, der mit je einer
Anschlagfläche (4a,13a,23a) an jeweils einem Hohlelement (1,2,11,20) zusammenwirkt.

8. Vorrichtung nach einem der Ansprüche 1 bis 6,
**dadurch gekennzeichnet, dass** das Verbindungselement (3,25) mit dem ersten Hohlelement (1,2,11,20) einstückig zusammenhängt oder durch Kleben oder Schweißen mit diesem verbunden ist.

9. Vorrichtung nach Anspruch 1 oder einem der folgenden, **dadurch gekennzeichnet, dass** das erste oder zweite Hohlelement (11) als Blutgefäßanschlussstück ausgebildet ist mit einem radial nach außen ragenden Anschlussflansch (11a) an seinem dem Schnappelement entgegengesetzten Ende.

10. Vorrichtung nach Anspruch 9, **dadurch gekennzeichnet, dass** der Anschlussflansch (lla) eine konvexe Anschlussfläche aufweist.

11. Vorrichtung nach Anspruch 10, **dadurch gekennzeichnet, dass** die Anschlussfläche in einer ersten Richtung gerade verläuft und in einer zweiten Richtung entsprechend der Außenkontur eines Blutgefäßes(9) einen runden, insbesondere teilkreisrunden Querschnitt aufweist.

12. Vorrichtung nach Anspruch 9, 10 oder 11, **dadurch gekennzeichnet, dass** das Blutgefäßanschlussstück (11) an seinem dem Anschlussflansch (lla) entgegengesetzten Ende eine kegelförmige Dichtfläche (11b) aufweist.

13. Vorrichtung nach Anspruch 9, 10 oder 11, **dadurch gekennzeichnet, dass** das Blutgefäßanschlussstück (11)an seinem dem Anschlussflansch (lla) entgegengesetzten Ende eine kugelflächenförmige Dichtfläche (23) aufweist.
